# EUROPEAN PATENT SPECIFICATION

(11) **EP 2 462 930 B1**
(45) Date of publication and mention of the grant of the patent: **31.05.2017**
(21) Application number: 12001416.2
(22) Date of filing: 02.11.2007
(51) Int. Cl.: A61K 31/12, A01K 43/04, A61K 31/715, A61K 39/12, A61K 39/295, C07K 14/18

(54) **Induction of an immune response against dengue virus using prime-boost approach**
Herbeiführung einer Immunreaktion gegen das Dengue-Virus mithilfe des Prime-Boost-Verfahrens
Induction d'une réponse immunitaire contre le virus de la dengue utilisant une approche de première immunisation

(30) Priority: 09.11.2006 US 860233 P
(43) Date of publication of application: 13.06.2012
(62) Divisional of application: 07874490.1
(73) Proprietor: The United States Of America As Represented By The Secretary Of The Navy, Silver Spring MD 20910 (US)
(72) Inventor: Simmons, Monika, Germantown, MD 20874 (US); Porter, Kevin R., Boyds, MD 20841 (US); Kanakatte, Raviprkash, Clarksville, MD 21029 (US); Sun, Wellington, Kensington, MD 20895 (US)
(74) Representative: Grosse Schumacher Knauer von Hirschhausen

(56) References cited:
- US-B1- 6 455 509
- KHANAM S ET AL: "Induction of neutralizing antibodies and T cell responses by dengue virus type 2 envelope domain III encoded by plasmid and adenoviral vectors", VACCINE, ELSEVIER LTD, GB LNKD- DOI:10.1016/J.VACCINE.2006.06.031, vol. 24, no. 42-43, 30 October 2006 (2006-10-30), pages 6513-6525, XP025151633, ISSN: 0264-410X [retrieved on 2006-10-30]
- SIMMONS MONIKA ET AL: "Characterization of antibody responses to combinations of a dengue virus type 2 DNA vaccine and two dengue virus type 2 protein Vaccines in rhesus macaques", JOURNAL OF VIROLOGY, vol. 80, no. 19, October 2006 (2006-10), pages 9577-9585, XP002588919, ISSN: 0022-538X
- KONISHI E ET AL: "Dengue tetravalent DNA vaccine inducing neutralizing antibody and anamnestic responses to four serotypes in mice", VACCINE, ELSEVIER LTD, GB LNKD- DOI:10.1016/J.VACCINE.2005.11.002, vol. 24, no. 12, 15 March 2006 (2006-03-15), pages 2200-2207, XP025151183, ISSN: 0264-410X [retrieved on 2006-03-15]
- MOTA J ET AL: "Induction of protective antibodies against dengue virus by tetravalent DNA immunization of mice with domain III of the envelope protein", VACCINE, ELSEVIER LTD, GB LNKD- DOI:10.1016/J.VACCINE.2004.12.028, vol. 23, no. 26, 16 May 2005 (2005-05-16), pages 3469-3476, XP004852137, ISSN: 0264-410X
- RAVIPRAKASH K ET AL: "Needle-free Biojector injection of a dengue virus type 1 DNA vaccine with human immunostimulatory sequences and the GM-CSF gene increases immunogenicity and protection from virus challenge in Aotus monkeys", VIROLOGY, ACADEMIC PRESS,ORLANDO, US LNKD- DOI:10.1016/S0042-6822(03)00542-7, vol. 315, no. 2, 25 October 2003 (2003-10-25), pages 345-352, XP004469556, ISSN: 0042-6822
- APT D ET AL: "Tetravalent neutralizing antibody response against four dengue serotypes by a single chimeric dengue envelope antigen", VACCINE, ELSEVIER LTD, GB LNKD- DOI:10.1016/J.VACCINE.2005.07.100, vol. 24, no. 3, 16 January 2006 (2006-01-16), pages 335-344, XP025151079, ISSN: 0264-410X [retrieved on 2006-01-16]
- CHEN LAN ET AL: "A heterologous DNA prime-Venezuelan equine encephalitis virus replicon particle boost dengue vaccine regimen affords complete protection from virus challenge in cynomolgus macaques.", JOURNAL OF VIROLOGY NOV 2007 LNKD- PUBMED:17715224, vol. 81, no. 21, November 2007 (2007-11), pages 11634-11639, XP002588805, ISSN: 0022-538X
- KEVIN R PORTER ET AL: "A dengue-VEE replicon particle vaccine induces the best immune response in a prime-boost regimen", AMERICAN JOURNAL OF TROPICAL MEDICINE & HYGIENE., vol. 73, no. 6, Suppl. S, 1 December 2005 (2005-12-01), - 15 December 2005 (2005-12-15), pages 180-181, XP055115849, US ISSN: 0002-9637
- Kevin P. Porter: "A dengue-VEE replicon particle vaccine induces the best immune response in a prime-boost regimen", The American Journal of Tropical Medicine and Hygiene, 11 December 2005 (2005-12-11), page 549, XP055312621, 54th Annual meeting of the American Society for Tropical Medicine and Hygiene, Washington Retrieved from the Internet: URL:http://www.ajtmh.org/content/73/6_supp l [retrieved on 2016-10-20]

## Description

### BACKGROUND OF INVENTION

### Field of Invention

The inventive subject matter relates to a prime-boost composition for use in a method of including a prophylactic immune response against dengue virus.

### Background

Dengue virus, the causative agent of dengue fever (DF) and dengue hemorrhagic fever (DHF), is a virus of the genus Flavivirus, a single-stranded enveloped RNA virus with positive polarity. Its RNA encodes approximately 3,400 amino acids. The virus exists as four antigenically-distinguishable serotypes.

Dengue fever is the most common human arbovirus infection worldwide and a serious public health concern accounting for estimates of 100 million infections annually (WHO 1986; Monath and Helnz 1996; Thomas, et al 2003). DF and DHF are found in most tropical areas including Africa, Asia, the Pacific, Australia, and the Americas,

Although the virus is capable of growing in a variety of species of mosquitoes, including Aedes albopictus, Aedes polynesiensis and Aedes scutellaris, Aedes aegypti is the most efficient mosquito vector because of Its domestic habitat (Gubler 1988). Four antigenically distinct serotypes of dengue virus have been Identified with all causing human diseases (Gubler, et al 1979; Henohal and Putnak 1990). Each of the four
serotypes, although distinct, is similar enough to the others to elicit only partial cross-protection following infection (WHO 1986). Following infection, viremia is typically detected early at the onset of symptoms (Halstead 1997). Although many dengue infections are mild, some infections result in DHF and dengue shock syndrome (DSS), which are potentially fatal. This usually occurs in a small number of people during a second infection caused by a dengue virus that is different from the virus causing the first infection (Halstead 1997).

Dengue virus infection occurs following the bite of dengue virus-infected *Aedes* mosquitoes, which were previously infected by feeding on infected humans. Symptoms of dengue infection include high fever, severe headache, retro-orbital pain, development of a rash, nausea, joint and muscle pain, and usually start within five to six days following the bite of an infected mosquito. Symptoms of DHF also include marked sub-dermal bleeding, causing a purplish bruise, as well as bleeding from the nose, gums, and gastrointestinal (GI) tract. The fatality rate associated with DHF is at 6 to 30% with most deaths occurring in infants. The management of DHF is symptomatic and supportive, and is aimed at replacement of fluid loss (Nimmannitya 1996).

It is not possible to make an accurate diagnosis of mild or classic DF based on clinical features alone since many symptoms of DF resemble those of other diseases, such as chikungunya infection (Nimmannitya 1996). measles, influenza, and rickettsial infections. Differential diagnosis must include malaria and other viral, bacterial, and rickettsial diseases. Diagnostic methods for infection are typically based on detection of virus, viral antigens, genomic sequences, and detection of dengue-specific antibodies (Shu and Huang 2004). DHF can, in some cases, be more accurately diagnosed based on clinical signs and symptoms, including high continuous fever for 2 to 7 days, hepatomegaly, hemoconcentration, shock and thromocytopenia.

Most infections result in DF, which is self-limiting. However, DHF and DSS are life-threatening. Although vaccines against other flaviviruses, such as yellow fever and Japanese encephalitis, have been licensed, there are currently no efficacious vaccines to protect against DF, DHF or DSS.

Two dengue tetravalent live-attenuated vaccine (TLAV) candidates currently exist However, both of these vaccines may be either reactogenic or poorly immunogenic in some recipients. Promising alternatives include chimeric viruses (e.g.. Yellow fever/Dengue), recombinant proteins, inactivated viruses and nucleic acid (DNA) vaccines. The DNA vaccines may be particularly useful at eliciting a cell-mediated as well as a humoral immune response.

Experimental evidence suggests that, in non-human primates, dengue DNA vaccines, given alone, require several booster administrations and long intervals between the administrations in order to induce protective immunity. Other non-replicating vaccines such as the purified inactivated vaccines can often induce high titers of neutralizing antibody but these vaccines may be poor inducers of long-term immunological memory. Therefore, a safe, efficacious immunization method and composition is needed for the more timely induction of long-lasting immunity to dengue virus infection.

Porter, K.R. et al. 2005. A dengue-VEE replicon particle vaccine induces the best immune response in a prime-boost regimen. Am. J. Trop. Med. Hyg. 73(6) suppl: 180-181 teaches a prime boost composition utilizing single strain viral elements, wherein enhancing the immune response to a dengue DNA vaccine by a prime-boost regimen utilizing a Venezuelan Equine Encephalitis Virus replicon system which expresses the pre-membrane and complete envelope protein or a truncated version thereof of DEN-1 West Pac 74 strain.

US Patent US 6,455,509 discloses dengue nucleic acid vaccines that induce neutralizing antibodies, wherein the vaccine may include a eukaryotic plasmid expression vector which includes at least part of the envelope gene and optionally the PreM gene of dengue virus.

### BRIEF SUMMARY OF INVENTION

The Invention relates to a prime-boost composition for use in a methods of inducing a prophylactic immune response against dengue (DEN) virus according to claim 1. The forthfollowing description also explains, for the sake of complete disclosure and exhaustive description and thus for better understanding, methods of inducing an immune response against dengue virus, e.g. method for the induction of immune response against dengue virus with reduced reactogencity by priming the subject with a non-replicating immunogen and boosting with a tetravalent live attenuated viral vaccine. Examples of non-replicating immunogens include tetravalent DNA vaccines containing DNA sequences encoding dengue virus proteins or tetravalent purified inactivated dengue virus protein vaccines.

Further described for the sake of completeness are methods of inducing an immune response to dengue virus via heterologous prime-boost vaccination regimens. The priming and boosting compositions contain different expression systems encoding and expressing dengue viral proteins. The expressions systems include adenoviral expression vectors, DNA expression vectors, and Venezuelan equine encephalltis virus replicon expression systems.

### BRIEF DESCRIPTION OF THE DRAWINGS

FIG. 1. Graphs illustrating serum immunoglobulin (IgG) antibody responses of non-human primates immunized with a tetravalent dengue DNA vaccine (TDNA), a tetravalent dengue purified inactivated vaccine (TPIV), followed by a tetravalent dengue live attenuated vaccine (TLAV). Samples were tested by ELISA at a 1:100 dilution.
FIG. 2. Bar graphs illustrating neutralizing antibody on day 60 following immunization of monkeys with a tetravalent dengue DNA vaccine (TDNA), a tetravalent dengue purified inactivated vaccine (TPIV), and a tetravalent dengue live attenuated vaccine (TLAV).

### DESCRIPTION OF PREFERRED EMBODIMENTS

### PRIME-BOOST METHOD USING DNA/TPIV/TLAV

A need exists for the induction of a long-lasting, efficacious immune response to dengue virus infection. To fulfill this critical need, the contemplated invention comprises immunizing methods that use dengue virus DNA or inactivated dengue virus proteins as a priming immunogen with live attenuated dengue virus as a boosting immunogen in a prime-boost immunization scheme (See Example 1 and 2). The resultant immune response has greater efficacy and safety.

Live attenuated viruses (LAV) often exhibit significantly elevated immune responses over other immune compositions, but also frequently exhibit detrimental reactogenicity. In order to improve the immunogenicity of anti-dengue vaccines, while reducing potential reactivity, an aspect contemplates a method for the induction of immunity to dengue virus comprising administering a tetravalent DNA vaccine (TDNA) or a tetravalent purified inactivated vaccine (SPIV) as a priming immunogen followed by a boost with a tetravalent live attenuated virus (TLAV). The inventive rationale is that priming with non-replicating vaccines, such as DNA or protein, will generate an immune response that will reduce reactogenicity and improve immunogenicity of the TLAV (See Example 1).

### HETROLOGOUS PRIME-BOOST METHOD USING VEE RFPLICON/DNA EXPRESSION VECTOR/ADENOVIRUS EXPRESSION VECTOR

The use of a Venezuelan equine encephalitis virus (VEE) replicon or adenovirus vector to express dengue virus proteins as either a prime or a boost, is contemplated which is described in detail in Example 3. In the contemplated immunization methods, either adenovirus expression vectors, DNA expression vector or VEE replicon, with each containing dengue virus DNA sequences comprise the prime immunization. Subsequent to the prime immunization, a heterologous boost is administered either as an adenovirus expression vector or VEE replicon, with each containing sequences coding for dengue virus proteins (See table 2).

### EXAMPLES

### Example 1: Composition and method of inducing anti-dengue response using TDNA/TPIV/TLAV

Groups of rhesus macaques (N=4) were primed with either two (2) doses of TDNA, one dose of TPIV, or one dose of TLAV, followed by boosting with TLAV. The dengue TLAV was made by serial passage of four wild-type manovalent virus isolates in primary dog kidney (PDK) cells. The passaged viruses were tested in rhesus monkeys where they induced significantly lower levels of viremia compared with unpassaged wild-type parent viruses. They were then propagated in fetal rhesus lung (FRhL) cells and combined to make the TLAV formulation. The formulation may comprise any combination of dengue virus strains and proteins can be utilized. A preferred aspect, illustrated in this example, consists of DEN 1 PDK 27, DEN 2 PDK 50, DEN 3 PDK 20 and DEN 4 PDK 6.

The TDNA can consist of DNA sequences or constructs encoding any dengue protein. A preferred aspect, is illustrated in this example, consists of the pre-membrane (prM) and envelope (E) genes of DEN 1 West Pac, DEN 2 wild-type/Phil + lysosome associated membrane protein (LAMP) domain, DEN 3 wild-type/Phil, and DEN 4 wild-type/Phil. The DEN 2 construct has a replacement of the C-terminal transmembrane and cytoplasmic domains of E with LAMP.

Similarly, TPIV can be a combination of one or more purified inactivated dengue virus proteins. As an illustration, in a preferred aspect, the TPIV consists of the core protein (C), pre-membrane (prM), envelope (E) and nonstructural protein 1 (NS1) of DEN 1 (West Pac), DEN 2 (S16803), DEN 3 (CH53489) and DEN 4 (TVP-360). The viruses were grown in Vero cells, purified, inactivated with formalin and adsorbed onto 0.1% aluminum hydroxide.

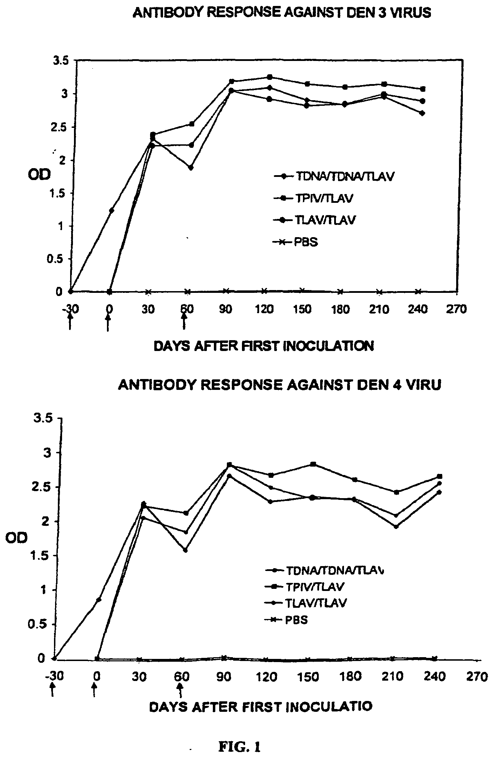

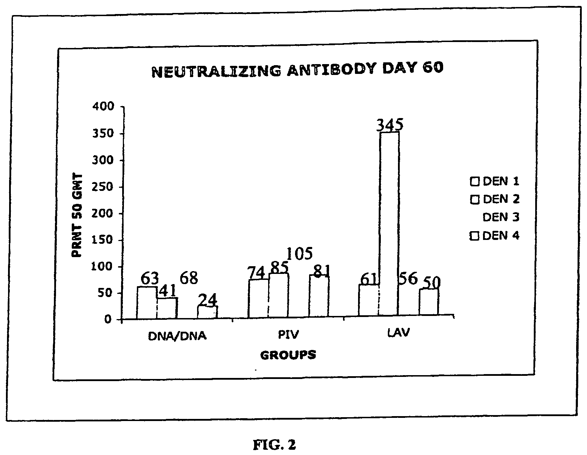

Referring to FIG. 1, antibody responses measured by ELISA demonstrated tetravalent immune responses and high titers of dengue-specific IgG in all groups, which were maintained until the day of challenge. Referring to FIG. 2, low-titered virus-neutralizing antibodies (Nab) were demonstrated against DEN-1, DEN-3 and DEN-4 after priming in all vaccine groups. Nab against DEN-2 were highest in animals that received the TLAV (GMT = 1216) followed by groups that received TPIV (GMT = 347) and TDNA (GMT =126). Nab titers peaked one month after the TLAV booster and then declined over time in all groups. The most persistent tetravalent Nab titers were observed with the TPIV/TLAV regimen.

Six months after the booster vaccination, all vaccinated animals and an unvaccinated control group were challenged with live, non-attenuated DEN-3 virus. As shown in Table 1, serum viremia was measured for 10 days after the live virus challenge to evaluate protection. Complete protection against viremia was observed in the TLAV/TLAV group and the TPIV/LAV group. Three of four animals in TDNA/TDNA/TLAV group exhibited 1 to 3 days of viremia (mean = 1.5 days) compared with unvaccinated controls, which had 4.75 mean days of viremia. Measurement of virus Nab titers 14 days after challenge showed a 2-5 fold and 2-10 fold increase in the TPIV/TLAV and TLAV/TLAV groups respectively, whereas the TDNA/TLAV regimen resulted in a 6-53 fold increase.

**Table 1**

| Viremia After Challenge | | | | | | | | | | | | |
|---|---|---|---|---|---|---|---|---|---|---|---|---|
| Group | Monkey | Days of viremia | | | | | | | | | | Mean days of viremia (for grp) |
| | | 1 | 2 | 3 | 4 | 5 | 6 | 7 | 8 | 9 | 10 | |
| DNA/DNA/LAV | A71 | - | - | - | - | - | - | + | - | - | - | |
| | 856Z | - | - | - | - | - | - | - | - | - | - | |
| | 894Z | + | - | + | - | - | - | - | - | - | - | |
| | 922Z | - | - | - | - | + | + | + | - | - | - | |
| | | | | | | | | | | | | 1.5 |
| PIV/LAV | 890Z | - | - | - | - | - | - | - | - | - | - | |
| | A63Z | - | - | - | - | - | - | - | - | - | - | |
| | 916Z | - | - | - | - | - | - | - | - | - | - | |
| | A96Z | - | - | - | - | - | - | - | - | - | - | |
| | | | | | | | | | | | | 0.0 |
| LAV/LAV | P146 | - | - | - | - | - | - | - | - | - | - | |
| | 860Z | - | - | - | - | - | - | - | - | - | - | |
| | 3158 | - | - | - | - | - | - | - | - | - | - | |
| | 928Z | - | - | - | - | - | - | - | - | - | - | |
| | | | | | | | | | | | | 0.0 |
| SAL/SAL | 914Z | + | + | + | + | + | + | - | - | - | - | |
| | B85 | - | + | - | - | - | - | + | - | + | + | |
| | 868Z | - | + | + | - | - | + | + | + | - | - | |
| | 898Z | - | + | + | + | + | - | - | - | - | - | |
| | | | | | | | | | | | | 4.75 |

The conclusion from these studies demonstrate that priming with TPIV resulted in increased vaccine immunogenicity and protective efficacy compared to priming with TDNA, and did not prevent effective boosting with TLAV.

### Comparative Example

### Example 2: Prophetic use of TDNA/TPIV/TLAV to induce human immunity

An aspect of the current invention is a method of administering TDNA/TPIV followed by TLAV formulation to humans in order to induce an anti-dengue immune response. The TDNA, TPIV and TLAV can be composed from any dengue gene sequence or strain, as illustrated in Example 1.

Other methods of administration may be used. However, as an illustration of the contemplated inventive method, the following prophetic example is disclosed as a preferred embodiment. In the prophetic example, the TDNA is administered intramuscularly as a total of 5 mg (1.25 mg/serotype) using the needle-free Biojector. The TDNA is given as two doses, one month apart. The TPIV is given as only one dose of 4 ug (1 ug/serotype) intramuscularly, using a needle and syringe. The TLAV is given as 5 logs/ serotype subcutaneously using a needle and syringe.

### Example 3: Prophetic examples of Prime/Boost Immunization using VEE replicon, adenovirus expression system, or DNA expression system compositions

The contemplated immunization method comprises a number of potential prime/boost compositions using VEE replicon, adenovirus expression system or a DNA expression system, each system containing dengue gene sequences. Referred compositions and combinations of prime-boost are illustrated in Table 2.

**Table 2: Combinations of prime-boost Compositions**

| **Prime** | **Boost** |
|---|---|
| DNA expression system | Adenovirus expression vector |
| VEE replicon system | Adenovirus expression vector |
| DNA expression system | VEE Replicon system |
| Adenovirus expression vector | VEE replicon system |

For example, the adenovirus expression vector, listed in Table 2 can be any adenoviral expression vector. The contemplated adenoviral expression vector has the E1 and E4 genes removed, which are replaced with the pre-membrane (prM) and envelope (E) genes from either DEN 1 and DEN 2 or DEN 3 and DEN 4. The contemplated adenoviral composition, therefore, is administered either as a single adenoviral vector expressing genes from only two dengue strains (i.e., DEN 1 and 2 or 3 and 4) or a mixture of 2 adenoviral vectors with one expressing DEN 1 and 2 and the other vector expressing DEN 3 and 4.

The DNA expression system is any suitable DNA expression system capable of *in vivo* expression. A preferred system is pVR1012 (see Patent No. 6,455,509 to Kochel, et al). In this composition, a DNA sequence or construct encoding dengue membrane and envelope genes for either DEN 1, 2, 3 or 4 are inserted into the plasmid. The composition, therefore, is either a single plasmid containing genes to a single dengue strain or a mixture of 2 or more plasmids with each containing genes from different strains of dengue.

Similar to the DNA expression system, the Venezuelan equine encephalitis (VEE) replicon system (VRP) contains pre-membrane and envelop proteins from either of DEN 1, 2, 3 or 4. Like the DNA vaccine composition, the VRP composition is either a single VRP containing genes to a single dengue strain or a mixture of 2 or more VRP systems with each containing genes from different strains of dengue. The dengue prM and E genes are cloned into a plasmid vector containing the VEE genome, replacing the VEE capsid and glycoprotein genes. This recombinant plasmid contains all the sequences (except the VEE capsid and glycoprotein) for packaging the RNA into replicon particles. Two other plasmids, one each containing the VEE capsid and the glycoprotein genes provide the missing elements in trans, and form parts of the tripartite (three-plasmid) system. RNA is prepared from each of the three plasmids by *in vitro* transcription.

A mixture of all 3 RNAs is used to transfect BHK (baby hamster kidney) cells. The RNAs are translated into proteins in the transfected cells. The transfected BHK cells then produce VRPs into which the recombinant RNA containing dengue genes has been packaged. These VRPs are purified and used as a vaccine. The dengue-VRP vaccines can infect cells but can not propagate new progeny.

### Example 4: vaccination using a DNA expression systemlVEE replicon prime/boost composition

As an illustration, a candidate vaccine DIME-VRP expressing dengue virus type 1 pre-membrane (prM) and envelope (E) proteins from a Venezuelan equine encephalitis virus (VEE) replicon system was constructed Three vaccination regimens (DIME DNA vaccine, DIME-VRP, and a heterologous prime-boost vaccine with DIME DNA as the prime immunogen and DDIME-VRP as the boost immunogen) were compared for immunogenicity and protection against dengue-1 virus challenge in a non-human primate model.

Groups of 3 and 4 cynomolgus macaques were immunized with three doses of DIME DNA vaccine (DDD), three doses of DIME-VRP (vvv) or with two doses of DNA priming vaccine and third booster dose of DIME-VRP (DDV). A control group of animals was inoculated with PBS. Virus neutralizing antibody was measured by plaque reduction neutralization test (PRNT) and 50% neutralization titers (PRNT-50) were determined by probit analysis. T cell responses were measured by gamma-IFN ELISPOT. Measured 4 weeks after final immunization, the DDV group produced the highest virus neutralizing antibody titers (PRNT-50 = 2304) followed by VVV (PRNT-50 = 1405) and DDD (PRNT-50 = 1364) groups. However, moderate T cell responses were demonstrated only in DDD and DDV vaccinated animals.

Five months after the final dose, all animals were challenged with live dengue-1 virus and viremia was determined by infecting Vero cells with sera collected from daily bleeds. All three (3) control animals became viremic for 6-7 days (mean = 6.3 days). All vaccination regimens showed significant protection from viremia. DDV immunized animals were completely protected from viremia (mean = 0 days). DDD and VW vaccinated animals had mean days of viremia of 0.66 and 0.75, respectfully. Thus, the antibody response and protection elicited from DlME-VRP was comparable to those elicited from DlME-DNA vaccine. However, the prime-boost approach resulted in higher antibody responses and complete protection.

### REFERENCES

1. Gubler, D.J., S. Nalim, R. Tan, H. Saipan, and J. Sulianti Saroso. 1979. Variation in susceptibility to oral infection with dengue viruses among geographic strains of Aedes aegypti. Am. J. Trop. Med. Hyg. 28:1045-1052.
2. Gubler, D.I. 1988. Dengue. In The Arboviruses: Epidemiology and Ecology. T.P. Monath (ed.), CRC Press (Boca Raton), p223-260.
3. Halstead, S.B. 1997. Epidemiology of dengue and dengue hemorrhagic fever. In Dengue and Dengue Hemorrhagic Fever. D.J. Gubler and G. Kuno, editors. Cab international, London. 23-44.
4. Henchal, E.A. and J.R. Putnak. 1990. The dengue viruses. Clin. Microbiol. Rev. 3:376-396.
5. Monath, T.P., and F.X. Heinz. 1996. Flaviviruses. In Fields Virology. B.N. Fields, D.M. Knipe and P.M. Howley, (eds.) Lippincott-Raven, Philadelphia. 961-1034.
6. Nimmannitya, S. 1996. Dengue and dengue haemorrhagic fever. In Manson's Tropical Diseases. G.C. Cook (eds.) W.B. Saunders Company, Ltd (London). 721-729.
7. Shu, P.Y., and J.H. Huang. 2004. Current advances in dengue diagnosis. Clin. Diagn. Lab. Immunol., 11(4):642-650.
8. World Health Organization. Dengue Hemorrhagic Fever. Diagnosis, Treatment and Control. Geneva: WHO,1986.

Further embodiments are:
1. A method of inducing an immune response to dengue virus comprising administering a priming dengue virus immunogen comprising a tetravalent DNA vaccine or a tetravalent purified inactivated vaccine and a boosting dengue virus immunogen comprising a tetravalent live attenuated viral vaccine.
2. The method of embodiment 1, wherein said tetravalent DNA vaccine contains DNA sequences encoding dengue proteins.
3. The method of embodiment 1, wherein said tetravalent DNA vaccine contains DNA sequence encoding the prM/E genes of DEN 1, DEN 2, DEN 3 and DEN 4.
4. The method of embodiment 3, wherein said DEN 2 DNA sequence is a construct that has a replacement of the C-terminal transmembrane and cytoplasmic domains of E with lysosome associated membrane domain.
5. The method of embodiment 1, wherein said tetravalent purified inactivated vaccine contains purified dengue proteins representing any strain of DEN 1, DEN 2, DEN 3 and DEN 4.
6. The method of embodiment 1, wherein said live attenuated viral vaccine comprises a live attenuated dengue virus representing any strain of DEN 1, DEN 2, DEN 3, and DEN 4.
7. The method of embodiment 1, wherein said boosting immunogen is administered between two weeks and 2 months of said administration of said priming immunogen.
8. A method of inducing an immune response to dengue virus comprising administering a priming dengue virus immunogen and boosting with a heterologous dengue virus immunogen wherein:
   a. said priming dengue virus immunogen is selected from the group consisting of:
      i) a DNA expression system;
      ii) an adenovirus expression vector; and
      iii) a Venezuelan equine encephalitis virus replicon system;
   b. said boosting dengue virus immunogen is selected from the group consisting of:
      i) an adenovirus expression vector; and
      ii) a Venezuelan equine encephalitis virus replicon system.
9. The method of embodiment 8, wherein said priming immunogen is composed of one or more adenovirus expression systems with each of said adenovirus systems containing DNA encoding pre-membrane and envelop genes from DEN 1 and DEN 2 or DEN 3 and DEN 4.
10. The method of embodiment 8, wherein said priming immunogen is composed of one or more DNA expression systems with each of said DNA systems containing DNA encoding dengue virus pre-membrane and envelop genes from dengue strains selected from the group consisting of DEN 1, DEN 2, DEN 3 and DEN 4.
11. The method of embodiment 8, wherein said priming immunogen is composed of one or more Venezuelan equine encephalitis virus replicon systems with each Venezuelan system containing DNA encoding dengue virus pre-membrane and envelop genes from dengue strains selected from the group consisting of DEN 1, DEN 2, DEN 3 and DEN 4.
12. The method of embodiment 8, wherein said boosting immunogen is composed of one or more adenovirus expression systems with each of said adenovirus system containing DNA encoding pre-membrane and envelop genes from both DEN 1 and DEN 2 or DEN 3 and DEN 4.
13. The method of embodiment 8, wherein said boosting immunogen is composed of one or more Venezuelan equine encephalitis virus replicon systems with each of said Venezuelan system containing DAN encoding dengue virus pre-membrane and envelop genes from dengue strains selected from the group consisting of DEN 1, DEN 2, DEN 3 and DEN 4.

## Claims

1. A prime-boost kit for use in a method of inducing a prophylactic immune response against dengue (DEN) virus, the prime-boost kit comprising two or more doses of a priming DEN virus immunogen to be administered consecutively, and one or more doses of a boosting DEN virus immunogen,
wherein the priming DEN virus immunogen is composed of a DNA expression system,
and wherein the boosting DEN virus immunogen is composed of a Venezuelan equine encephalitis virus replicon (VRP) system, wherein the VRP system and the DNA expression system contain DNA encoding DEN virus pre-membrane and envelope genes from DEN strains selected from the group consisting of DEN1, DEN2, DEN3 or DEN4,
wherein capsid and glycoprotein genes of the VRP system are replaced by pre-membrane and envelope genes of the same DEN serotype as in the DNA expression system, wherein RNA encoding the VRP system and RNA encoding the Venezuelan equine encephalitis glycoprotein or capsid gene are expressed together, and wherein VRP particles are purified and used in the vaccine.

2. Two or more prime-boost kits as defined by claim 1 for use in a method of inducing a prophylactic immune response against DEN virus.

3. One or more prime-boost kits for use according to any of claims 1 or 2, wherein said DNA expressing system is pVR1012.

## Patentansprüche

1. Prime-Boost-Kit zur Verwendung in einem Verfahren zum Herbeiführen einer prophylaktischen Immunantwort gegen ein Dengue (DEN)-Virus, wobei das Prime-Boost-Kit zwei oder mehr Dosen eines zum Priming verwendeten DEN-Virus-Immunogenes umfasst, die hintereinander zu verabreichen sind, sowie eine oder mehrere Dosen eines zum Boosting verwendeten DEN-Virus-Immunogens,
wobei das zum Priming verwendete DEN-Virus-Immunogen aus einem DNA-Expressionssystem zusammengesetzt ist
und wobei das zum Boosting verwendete DEN-Virus-Immunogen aus einem VRP-System (einem auf einem Replikon des venezuelanischen Pferdeenzephalitisvirus basierenden System) zusammengesetzt ist,
wobei das VRP-System und das DNA-Expressionssystem DNA enthalten, welche Prämembran- und Hüllgene des DEN-Virus aus DEN-Stämmen kodiert, die aus der Gruppe bestehend aus DEN1, DEN2, DEN3 oder DEN4 ausgewählt sind,
wobei Capsid- und Glycoproteingene des VRP-Systems durch Prämembran- und Hüllgene desselben DEN-Serotyps wie in dem DNA-Expressionssystem ersetzt sind,
wobei DNA, welche das VRP-System kodiert, und RNA, welche das Glycoprotein- oder Kapsidgen des venezuelanischen Pferdeenzephalitisvirus kodiert, zusammen exprimiert werden,
und wobei VRP-Partikel gereinigt und in dem Impfstoff verwendet werden.

2. Zwei oder mehr Prime-Boost-Kits nach der Definition in Anspruch 1 zur Verwendung in einem Verfahren zum Herbeiführen einer prophylaktischen Immunantwort gegen ein DEN-Virus.

3. Eines oder mehrere Prime-Boost-Kits zur Verwendung nach einem der Ansprüche 1 oder 2, wobei es sich bei dem DNA-Expressionssystem um pVR1012 handelt.

## Revendications

1. Kit de primovaccination-rappel pour son utilisation dans un procédé d'induction d'une réponse immunitaire prophylactique contre le virus de la dengue (DEN), le kit de primovaccination-rappel comprenant deux doses ou plus d'un immunogène du virus DEN de primovaccination à administrer de manière consécutive, et une dose ou plus d'un immunogène du virus DEN de rappel, dans lequel l'immunogène du virus DEN de primovaccination se compose d'un système d'expression d'ADN, et dans lequel l'immunogène du virus DEN de rappel se compose d'un système de réplicon du virus de l'encéphalite équine du Venezuela (VRP),
dans lequel le système VRP et le système d'expression d'ADN contiennent de l'ADN codant pour les gènes pré-membranaire et d'enveloppe du virus DEN de souches de DEN sélectionnées dans le groupe constitué de DEN1, DEN2, DEN3 ou DEN4,
dans lequel les gènes de capside et de glycoprotéine du système VRP sont remplacés par les gènes pré-membranaire et d'enveloppe du même sérotype de DEN que dans le système d'expression d'ADN,
dans lequel l'ARN codant pour le système VRP et l'ARN codant pour le gène de glycoprotéine ou de capside de l'encéphalite équine du Venezuela sont exprimés ensemble, et dans lequel les particules de VRP sont purifiées et utilisées dans le vaccin.

2. Deux kits de primovaccination-rappel ou plus selon la revendication 1, pour leur utilisation dans un procédé d'induction d'une réponse immunitaire prophylactique contre le virus DEN.

3. Un kit de primovaccination-rappel ou plus selon l'une quelconque des revendications 1 ou 2, dans lequel lendit système d'expression d'ADN est pVR1012.
